# EUROPÄISCHE PATENTSCHRIFT

(11) **EP 1 306 106 B1**
(45) Veröffentlichungstag und Bekanntmachung des Hinweises auf die Patenterteilung: **26.03.2008**
(21) Anmeldenummer: 02022555.3
(22) Anmeldetag: 08.10.2002
(51) Int. Cl.: A61N 5/06

(54) **Bräunungsmodul mit einem Gehäuse**
Tanning module with a housing
Module de bronzage avec un boitier

(30) Priorität: 24.10.2001 DE 10151850
(43) Veröffentlichungstag der Anmeldung: 02.05.2003
(73) Patentinhaber: Heraeus Noblelight GmbH, 63450 Hanau (DE)
(72) Erfinder: Ullrich, Bernd, 63526 Erlensee (DE); Berger, Ulrich, 63599 Biebergemünd (DE)
(74) Vertreter: Kühn, Hans-Christian

(56) Entgegenhaltungen:
- DE-A- 2 941 467
- DE-A- 4 323 936
- DE-U- 9 210 776
- FR-A- 711 054

## Beschreibung

Die Erfindung betrifft ein Bräunungsmodul mit einem Gehäuse, einem im Gehäuse angeordneten drei-dimensionalen Reflektor sowie mindestens einem scheibenförmigen Strahlungsfilter, wobei der mindestens eine Strahlungsfilter die Strahlungsaustrittsfläche des Reflektors überdeckt und an einer ersten Seite des Gehäuses angeordnet ist, wobei im Reflektor mindestens eine Öffnung zum Einbau und elektrischen Anschluss eines Bräunungsstrahlers vorgesehen ist, und wobei der Reflektor im Bereich der Strahlungsaustrittsfläche seinen maximalen Querschnitt aufweist.

Derartige Bräunungsmodule sind beispielsweise aus der DE 29 41 467 A1 bekannt, wobei ein rechteckiges Gehäuse inklusive Gebläse und Strömungsleitplatten im Gehäuse eingesetzt werden. Das rechteckige Gehäuse ist dabei in eine Luftansaugseite und eine Druckseite geteilt, wobei der Bräunungsstrahler auf der Druckseite angeordnet ist und indirekt gekühlt wird. Die Einsaugung von Kühlluft, welche entlang der Filterscheibe strömt und diese kühlt, erfolgt auf der Luftansaugseite des Gehäuses. Aus der Druckseite des Gehäuses verlässt die Kühlluft das Gehäuse durch die gleiche Seitenwand, wie sie zuvor auf der Luftansaugseite in das Gehäuse gelangt ist.

Die DE 195 16 603 A1 offenbart ein Niederdruckgesichtsfeld für Bräunungsgeräte, wobei ein rechteckiges Gehäuse inklusive Reflektor und Filterscheibe zum Einsatz kommt. Das Gehäuse ist zum Einbau mehrerer UVC-Röhren geeignet. Der Einlass von Kühlluft in das Gehäuse erfolgt über eine Öffnung zwischen Gehäuse und Filterscheibe, wobei die Kühlluft entlang der Röhren strömt und auf der Gehäuserückseite das Gehäuse wieder verlässt.

Die DE 36 31 427 C2 beschreibt ein Bestrahlungsgerät mit einem rechteckigen Gehäuse und Reflektor sowie Filterscheibe. Es handelt sich hier um ein Gerät niedriger Leistung, so dass eine Kühlung des Bräunungsstrahlers bzw. des Reflektors nicht vorgesehen ist. Zur Sicherung des Filters gegen Bruch ist ein Druckschalter vorgesehen, der von der Filterplatte in seiner eingedrückten Stellung festgesetzt wird, jedoch bei Bruch aus dieser Stellung heraustritt und die Strahlungsquelle abschaltet.

Die DE 39 27 695 C2 offenbart ein Bräunungsgerät mit einem schwenkbar angeordneten Interferenzfilter. In Strahlenaustrittsrichtung ist dem Interferenzfilter ein Infrarotfilter nachgeordnet. Je nach Neigung des Interferenzfilters im Strahlenaustritt wird die Grenze des Transmissionsspektrums zum kurzwelligeren UV-B-Anteil oder zum langwelligeren UV-A-Anteil verschoben. Damit ist durch das Verschwenken des Filters das Strahlungsspektrum auf den Hauttyp der zu bestrahlenden Person einstellbar.

Die DE 40 37 483 C2 beschreibt ein UV-Bestrahlungsgerät mit Bruchsicherung für eine Filterglasscheibe, wobei an deren Umfang eine stromdurchflossene elektrische Leiterbahn angeordnet ist. Bei Bruch der Filterglasscheibe wird die Leiterbahn und damit der Strom unterbrochen und der Bräunungsstrahler abgeschaltet.

Die DE 43 23 936 A1 beschreibt ein Gerät zur Ganzkörperbestrahlung, dessen besonderes Konstruktionsmerkmal im Wesentlichen darin besteht, dass das den Reflektor sowie die IR- und UV-Strahler enthaltende Lampengehäuse mittels einer speziellen Halterung und Transportvorrichtung mit gleichmäßiger Geschwindigkeit über die Gesamtlänge des zu bestrahlenden Körpers hin und her bewegt wird.

Es stellt sich das Problem, ein Bräunungsmodul zur Verfügung zu stellen, das ein Gehäuse mit optimierter Strömungsführung der Kühlluft aufweist.

Das Problem wird dadurch gelöst, dass das Gehäuse an einer dem Strahlungsfilter entgegengesetzten zweiten Seite in Form eines Walmdaches oder eines Krüppelwalmdaches ausgestaltet ist, wobei der Dachfirst abgeflacht ist und vom Strahlungsfilter wegzeigt.

Eine solche Gehäuseform hat den Vorteil, dass der freie Querschnitt zwischen der Außenseite des Reflektors und der Innenseite des Gehäuses klein gehalten werden kann, so dass bei einem Absaugen von Luft aus dem Bereich hinter dem Reflektor eine verhältnismäßig gleichmäßige Strömungsverteilung entsteht. Die Temperatur des Reflektors wird dadurch vergleichmäßigt. Auf zusätzliche Strömungsleitbleche kann im erfindungsgemäßen Bräunungsmodul verzichtet werden, da über die Dachformen die Bildung von strömungstechnisch toten Ecken" vermieden werden kann. Im Bereich der Öffnung des Reflektors, die zum Einbau und Anschluss eines Bräunungsstrahlers dient, kann zudem Luft aus dem Reflektorraum gezogen und damit der Bräunungsstrahler direkt gekühlt werden.

Das erfindungsgemäße Bräunungsmodul ist für Bräunungsstrahler mit einer elektrischen Leistung im Bereich von 250W bis 1000W geeignet. Es ist hervorragend in den unterschiedlichsten Bräunungsgeräten oder medizinischen Apparaturen zur Bestrahlung des Gesichtsfeldes, des Dekolletebereichs, des Körperbereichs, des Bein- oder Fußbereichs einsetzbar. Auch der Einbau in Zimmerdecken, beispielsweise zur Bestrahlung von Liegeflächen aus größeren Distanzen, ist möglich. Das erfindungsgemäße Bräunungsmodul kann grundsätzlich in jeder Art von Halterung montiert werden, so dass es in alle Richtungen einer virtuellen Kugel strahlen kann. Mehrere Bräunungsmodule können neben- beziehungsweise nacheinander montiert werden, um ein größeres Bestrahlungsfeld zu erzeugen. Dabei besteht die Möglichkeit, pro Bräunungsmodul mit einen separaten Lüfter zu arbeiten oder aber mehrere Bräunungsmodule mit einem zentralen Lüfter zu betreiben.

Der Strahlungsfilter ist vorzugsweise parallel zur Strahlungsaustrittsfläche des Reflektors ausgerichtet.

Der abgeflachte Dachfirst kann durch einen planen Gehäusewandungsteil gebildet sein, wobei dieser parallel zum Strahlungsfilter ausgerichtet sein kann. Der abgeflachte Dachfirst kann aber auch durch einen gewölbten Gehäusewandungsteil gebildet sein, wobei dieser im Hinblick auf den Strahlungsfilter konkav oder konvex gewölbt ausgestaltet sein kann. Es hat sich insbesondere bewährt, wenn an das Walmdach oder das Krüppelwalmdach in Richtung des Strahlungsfilters ein rechteckiger Gehäusewandungsbereich anschließt. In diesem kann beispielsweise die Aufnahme des Strahlungsfilters erfolgen.

Besonders bevorzugt ist ein becher- oder wannenförmig ausgebildeter Reflektor, wobei der Becher oder Wannenboden des Reflektors entweder gewölbt oder planparallel zu dem mindestens einen Strahlungsfilter ausgeführt sein kann. Ein Umfang des Reflektors parallel zur Strahlungsaustrittsfläche beschreibt vorzugsweise einen Kreis, eine Ellipse, ein Rechteck oder ein Vieleck. Dabei ist es bevorzugt, wenn der Reflektor aus Facetten gebildet ist und der Umfang des Reflektors parallel zur Strahlungsaustrittsfläche ein Vieleck mit zwölf Ecken beschreibt.

Besonders geeignet ist dabei ein Reflektor, der eine Höhe im Bereich von 90 mm bis 95 mm aufweist und insbesondere 93,6 mm hoch ist. Das Zwölfeck in der Ebene der Strahlungsaustrittsfläche weist vorzugsweise einen maximalen Durchmesser (von Ecke zu Ecke) im Bereich von 210 mm bis 230 mm auf und beträgt insbesondere 210 mm.

Ein weiterer geeigneter Reflektor weist eine Höhe im Bereich von 110 mm bis 125 mm auf und ist insbesondere 118,7 mm hoch. Das Zwölfeck in der Ebene der Strahlungsaustrittsfläche weist vorzugsweise einen maximalen Durchmesser (von Ecke zu Ecke) im Bereich von 170 mm bis 200 mm auf und beträgt insbesondere 184 mm.

Ein weiterer geeigneter Reflektor weist eine Höhe im Bereich von 75 mm bis 90 mm auf und ist insbesondere 83,3 mm hoch. Das Zwölfeck in der Ebene der Strahlungsaustrittsfläche weist vorzugsweise einen maximalen Durchmesser (von Ecke zu Ecke) im Bereich von 205 mm bis 235 mm auf und beträgt insbesondere 220 mm.

Die Führung der Kühlluftströmung im Gehäuse ist insbesondere dann optimiert, wenn mindestens eine Luftabsaugöffnung im Bereich der Dachschrägen vorhanden ist. Dabei kann an der Luftabsaugöffnung ein Flansch angebracht sein. Zur Absaugung von Kühlluft aus dem Gehäuse kann an dem Flansch ein Luftabsaugschlauch angeordnet sein. Über einen solchen Luftabsaugschlauch ist eine zentrale oder dezentrale Luftabsaugung möglich.

Um die Saugleistung für das Bräunungsmodul einstellen zu können, kann eine Reduzierscheibe zur Verkleinerung der Luftabsaugöffnung vorhanden sein.

Dies ist beispielsweise dann sinnvoll, wenn mehrere Bräunungsmodule eingesetzt werden und diese unterschiedlich weit von einem zentralen, d.h. von allen Bräunungsmodulen gleichzeitig genutzten Lüfter entfernt sind oder die Luftabsaugung in einer Art Reihenschaltung von einem Bräunungsmodul über mindestens ein weiteres zum Lüfter erfolgt.

Außen am Gehäuse ist vorzugsweise mindestens eine Halterung für elektrische Anschlüsse oder Komponenten angeordnet, wobei als Komponenten, hier beispielsweise ein Zündgerät, ein Drucktaster oder eine Erdungslasche in Frage kommen.

Besonders bevorzugt ist es, zwischen Gehäuse und Reflektor eine Ansaugplatte anzuordnen, wobei die Strahlungsaustrittsfläche des Reflektors zur Ebene der Ansaugplatte nach oben oder unten verschoben ist, wobei mindestens eine Ansaugöffnung zwischen Ansaugplatte und Reflektor ausgebildet ist und die Ansaugplatte eine Aussparung für den Reflektor aufweist, die in der senkrechten Projektion auf den mindestens einen Strahlungsfilter die Größe der Strahlungsaustrittsfläche des Reflektors aufweist.

Durch diese Anordnung des Reflektors im bezug auf die Ansaugplatte wird vorzugsweise eine ringförmige Ansaugöffnung zwischen Ansaugplatte und Reflektor ausgebildet, durch welche Kühlluft aus dem Bereich zwischen Strahlungsfilter und Strahlungsaustrittsfläche des Reflektors in Richtung der Außenwand des Reflektors abgezogen werden kann. Es können aber auch mehrere, vorzugsweise ringförmig angeordnete Ansaugöffnungen zwischen Ansaugplatte und Reflektor ausgebildet sein, wobei eine möglichst symmetrische Ausrichtung der Ansaugöffnungen sich positiv auf die Gleichmäßigkeit der Kühlung des Reflektors und des Strahlungsfilters auswirkt. Die Temperaturverteilung am Strahlungsfilter hat einen entscheidenden Einfluss auf seine Transmissionseigenschaften.

Außerdem hat es sich bewährt, wenn eine Ansaugplatte das Gehäuse und den Reflektor allseitig im Bereich der Strahlungsaustrittsfläche des Reflektors verbindet, wobei die Ansaugplatte mindestens eine Ansaugöffnung aufweist und zudem eine Aussparung für den Reflektor aufweist, die in der senkrechten Projektion auf den mindestens einen Strahlungsfilter die Größe der Strahlungsaustrittsfläche des Reflektors aufweist.

Insbesondere weist die Ansaugplatte einen rechteckigen Umfang auf, wobei der Umfang des Reflektors parallel zur Strahlungsaustrittsfläche einen Kreis, eine Ellipse oder ein Vieleck beschreibt und wobei die mindestens eine Ansaugöffnung im Bereich einer Ecke der Ansaugplatte angeordnet ist.

Bevorzugt ist, dass vier Ansaugöffnungen in der Ansaugplatte ausgebildet sind und dass je eine Ansaugöffnung in je einer Ecke der Ansaugplatte angeordnet ist.

Dabei hat es sich als strömungstechnisch günstig erwiesen, wenn die mindestens eine Ansaugöffnung längs der Seiten der Ansaugplatte vergrößert ist.

Die Ansaugöffnung kann dazu beispielsweise trapezförmig sein, wobei die lange Seite des Trapezes zum Reflektor zeigt. Die lange Seite des Trapezes sowie deren gegenüberliegende Seite können gekrümmt sein.

Es hat sich bewährt, den Reflektor nur über die Ansaugplatte am Gehäuse zu befestigen. Dabei können zwischen Ansaugplatte und Reflektor auch diverse Abstandshalter vorgesehen sein.

Der mindestens eine Strahlungsfilter ist vorzugsweise über einen Schwenkmechanismus vom Gehäuse lösbar ist. Dabei soll der Schwenkmechanismus ein Verkippen des Strahlungsfilters im Hinblick auf das Gehäuse ermöglichen, wobei das Lösen des Strahlungsfilters vom Gehäuse erst nach einer Verschiebung des verkippten Strahlungsfilters im Gehäuse möglich sein soll. Dadurch wird ein benutzerfreundlicher Austausch des Strahlungsfilters möglich und auch ein plötzliches Herabfallen des Strahlungsfilters vermieden, da über einen solchen Schwenkmechanismus ein Herabfallen des Strahlungsfilters und damit ein Bruch wirkungsvoll verhindert werden kann.

Besonders einfach gestaltet sich die Anbringung des Strahlungsfilters am Gehäuse, wenn der mindestens eine Strahlungsfilter rechteckig ausgebildet ist.

Dabei wird der mindestens eine Strahlungsfilter mit einer Länge und einer Breite im Bereich von 215mm bis 240mm bevorzugt. Insbesondere weist der mindestens eine Strahlungsfilter eine Länge von 230mm und eine Breite von 225mm auf.

Der mindestens eine Strahlungsfilter ist vorzugsweise ein Interferenzfilter, da mit diesem der UV-Anteil dosiert werden kann und der sichtbare Teil des Lichts unterdrückt werden kann.

Es ist optimal, wenn mindestens eine Luftansaugöffnung zwischen dem mindestens einen Strahlungsfilter und dem Gehäuse vorhanden ist und/oder wenn mindestens eine Luftansaugöffnung zwischen dem mindestens einen Strahlungsfilter und dem Reflektor im Gehäuse vorhanden ist. Dabei ist allerdings darauf zu achten, dass kein ungefilterter Strahlungsanteil das Gehäuse über die Luftansaugöffnung verlässt. So kann Kühlluft über die Luftansaugöffnung in den Bereich zwischen Strahlungsfilter und Strahlungsaustrittsfläche des Reflektors strömen und von dort über Absaugöffnungen zur Außenseite des Reflektors abgezogen werden.

Es wird eine Ausführungsform des Bräunungsmoduls bevorzugt, bei welcher ein erster Strahlungsfilter und planparallel dazu ein zweiter Strahlungsfilter vorhanden ist, wobei der zweite Strahlungsfilter zwischen der Strahlungsaustrittsfläche des Reflektors und dem ersten Strahlungsfilter angeordnet ist und wobei der erste Strahlungsfilter der Interferenzfilter ist.

Der zweite Strahlungsfilter ist dann vorzugsweise ein UV-Filter oder ein Infrarotfilter.

Zur Bruchsicherung des mindestens einen Strahlungsfilters kann mindestens ein Drucktaster am Gehäuse angeordnet sein, der auf den mindestens einen Strahlungsfilter aufsetzt.

Dabei kann der Drucktaster senkrecht zur Strahlungsaustrittsfläche des Reflektors durch den Reflektor geführt werden oder aber senkrecht zur Strahlungsaustrittsfläche des Reflektors durch die Ansaugplatte geführt sein. Der Drucktaster kann aber auch an der Ansaugplatte angeordnet sein und auf den mindestens einen Strahlungsfilter aufsetzen. Bei einem Bruch des überwachten Strahlungsfilters wird über die Positionsänderung des Drucktasters die Stromzufuhr des Bräunungsstrahlers unterbrochen und der Benutzer vor einer unkontrollierten Strahlendosis geschützt.

Bräunungsmodul nach einem der Ansprüche 1 bis 39, **dadurch gekennzeichnet, dass** ein Sockel (13) im Bereich der Öffnung des Reflektors (3a, 3b) zum mechanischen und elektrischen Anschluss eines Bräunungsstrahlers vorgesehen ist.

Bräunungsmodul nach einem der Ansprüche 21 bis 40, **dadurch gekennzeichnet, dass** zwischen dem mindestens einen Strahlungsfilter (2a, 2b) und der Ansaugplatte (10) eine Abdeckplatte angeordnet ist, die von der Ansaugplatte (10) beabstandet angeordnet ist und welche eine Aussparung aufweist, die in der senkrechten Projektion auf den mindestens einen Strahlungsfilter die Größe der Strahlungsaustrittsfläche des Reflektors (3a, 3b) aufweist.

Im Bereich der Öffnung des Reflektors ist zum mechanischen und elektrischen Anschluss eines Bräunungsstrahlers vorzugsweise ein Sockel vorgesehen.

Zwischen dem mindestens einen Strahlungsfilter und der Ansaugplatte kann eine Abdeckplatte angeordnet sein, die von der Ansaugplatte beabstandet angeordnet ist und welche eine Aussparung aufweist, die in der senkrechten Projektion auf den mindestens einen Strahlungsfilter die Größe der Strahlungsaustrittsfläche des Reflektors aufweist. Diese Abdeckplatte verdeckt gegebenenfalls in der Ansaugplatte vorhandene Ansaugöffnungen für Kühlluft vor dem Auge des Benutzers.

Es hat sich insbesondere bewährt, wenn das Gehäuse im Bereich des Strahlungsfilters rechteckig ist und an allen vier Seiten mit jeweils einem Falz abschließt, wobei die vier Falze parallel zum Strahlungsfilter ausgerichtet sind. Dabei können zwei sich gegenüberliegende Falze der vier Falze zum Strahlungsfilter zeigen und die übrigen zwei sich gegenüberliegenden Falze der vier Falze vom Strahlungsfilter wegzeigen. Eine solche Ausgestaltung ist dann besonders zu empfehlen, wenn mehrere Bräunungsmodule nebeneinander in einem Bräunungsgerät eingesetzt werden sollen, so dass insbesondere die Falze, die vom Strahlungsfilter wegzeigen, im Übergangsbereich zwischen zwei Bräunungsmodulen die Sicht auf den rückseitigen Bereich des Bräunungsmoduls versperren.

Die Figurendarstellungen 1 bis 9 sollen das erfindungsgemäße Bräunungsmodul beispielhaft erläutern. Dabei zeigt
- Figur 1: das Gehäuse eines Bräunungsmoduls in dreidimensionaler Ansicht,
- Figur 2: ein Bräunungsmodul mit einem Gehäuse gemäß Figur 1 im Schnitt quer zum Dachfirst,
- Figur 3: ein Bräunungsmodul mit einem Gehäuse im Schnitt,
- Figur 4: ein Bräunungsmodul mit einem Gehäuse gemäß Figur 1 im Schnitt längs des Dachfirsts,
- Figur 5: ein weiteres Bräunungsmodul mit einem Gehäuse gemäß Figur 1 im Schnitt längs des Dachfirsts,
- Figur 6: eine Ansaugplatte inklusive vier Ansaugöffnungen,
- Figur 7: drei Bräunungsmodule im Schnitt,
- Figur8: eine dreidimensionale Ansicht mehrerer Bräunungsmodule

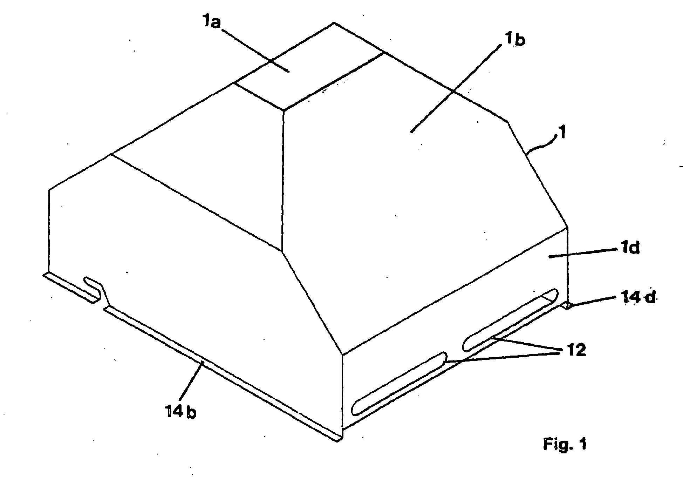

So zeigt Figur 1 das Gehäuse 1 eines Bräunungsmoduls in dreidimensionaler Ansicht, wobei hier die Form eines Krüppelwalmdaches 1b mit abgeflachtem Dachfirst 1a vorliegt. An die Dachkonstruktion schließt ein rechteckiger Gehäusewandungsbereich 1d an, der in vier Falzen endet, wobei zwei Falze 14b, 14d nach außen zeigen und die beiden übrigen Falze (hier nicht erkennbar) nach innen zeigen. Im rechteckigen Gehäusewandungsbereich 1d sind Luftansaugöffnungen 12 vorhanden.

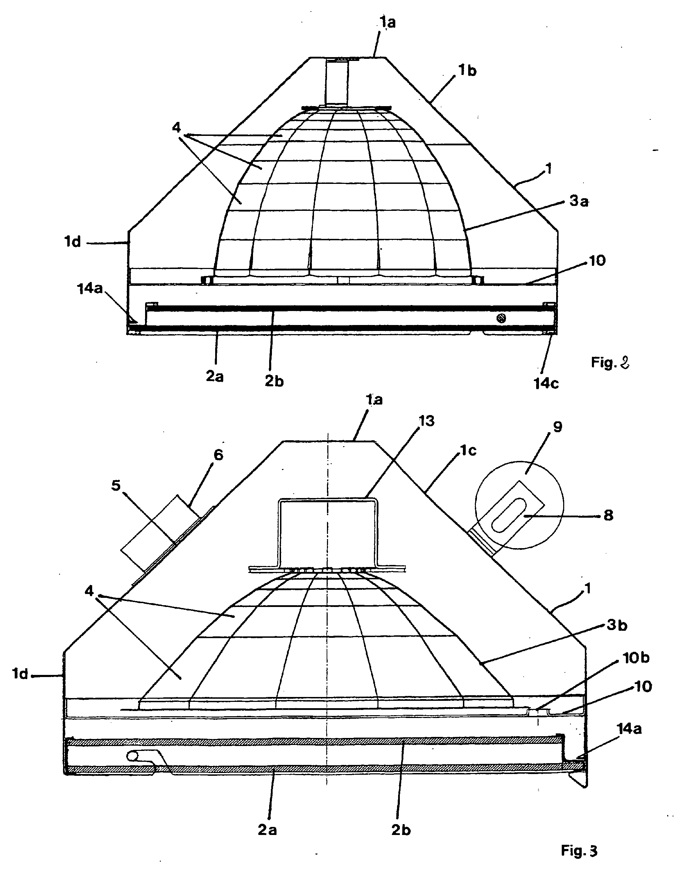

Figur 2 zeigt ein Bräunungsmodul mit einem Gehäuse 1 gemäß Figur 1 im Schnitt quer zum Dachfirst, wobei ein aus Facetten 4 gebildeter Reflektor 3a vorhanden ist. Der Reflektor 3a ist über Abstandshalter mit einer Ansaugplatte 10 verbunden, wobei Kühlluft zwischen den Abstandshaltern in den Raum zwischen Reflektor 3a und Gehäuse 1 gesaugt werden kann. Im rechteckigen Gehäusewandungsbereich 1d sind ein rechteckiger erster Strahlungsfilter 2a und ein rechteckiger zweiter Strahlungsfilter 2b vorhanden. Außerdem sind die Falze 14a, 14c kennbar, die zu den Strahlungsfiltem zeigen.

Figur 3 zeigt ein Bräunungsmodul mit einem Gehäuse im Schnitt quer zum Dachfirst, wobei ein aus Facetten 4 gebildeter Reflektor 3b vorhanden ist. Der Reflektor 3b ist mit einer Ansaugplatte 10 verbunden, wobei Kühlluft über Ansaugöffnungen in den Raum zwischen Reflektor 3b und Gehäuse 1 gesaugt werden kann. Außerdem ist eine Öffnung 10b mit Gewindedurchzug vorhanden, durch welche ein Drucktaster auf einen Strahlungsfilter aufsetzen kann. Im rechteckigen Gehäusewandungsbereich 1d sind ein rechteckiger erster Strahlungsfilter 2a und ein rechteckiger zweiter Strahlungsfilter 2b vorhanden. Außerdem ist der Sockel 13 zur Aufnahme eines Bräunungsstrahlers in der Öffnung des Reflektors 3b erkennbar. In der Dachschräge ist eine Luftabsaugöffnung 5 vorhanden, an der ein Flansch 6 angebracht ist. Außerdem ist ein Halter 8 zur Befestigung eines Zündgerätes 9 vorhanden.

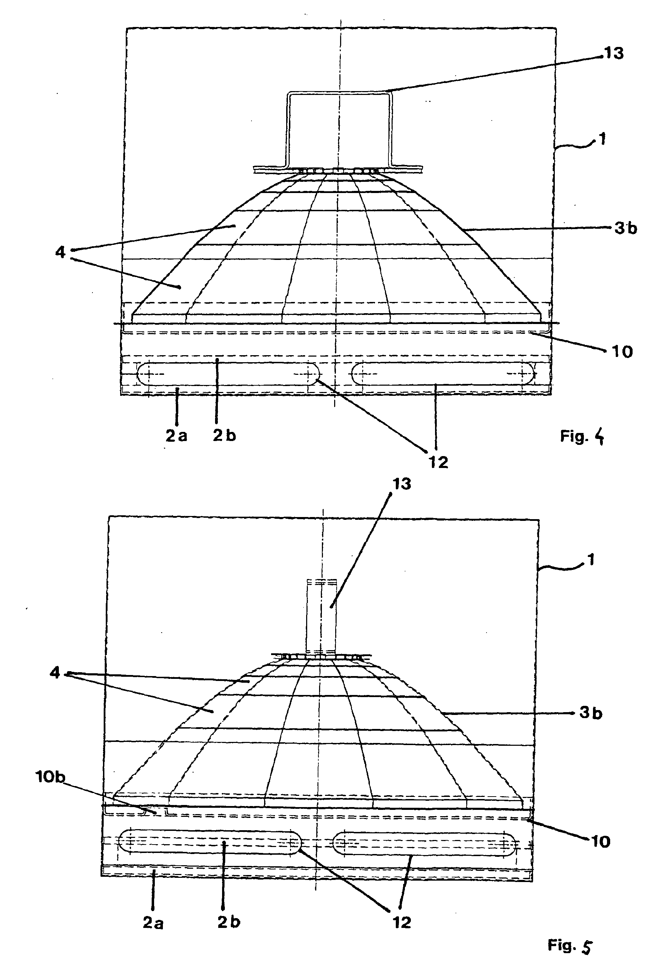

Figur 4 zeigt ein weiteres Bräunungsmodul mit einem Gehäuse 1 gemäß Figur 1 im Schnitt längs des Dachfirsts. Der Reflektor 3b ist beabstandet von der Ansaugplatte 10 mit dem Gehäuse 1 verbunden, wobei Kühlluft zwischen der Ansaugplatte 10 und dem Reflektor 3b in den Raum zwischen Reflektor 3b und Gehäuse 1 gesaugt werden kann.

Figur 5 zeigt ein weiteres Bräunungsmodul mit einem Gehäuse im Schnitt längs des Dachfirsts. Der Reflektor 3b ist mit der Ansaugplatte 10 verbunden, wobei Kühlluft über Ansaugöffnungen in den Raum zwischen Reflektor 3b und Gehäuse 1 gesaugt werden kann.

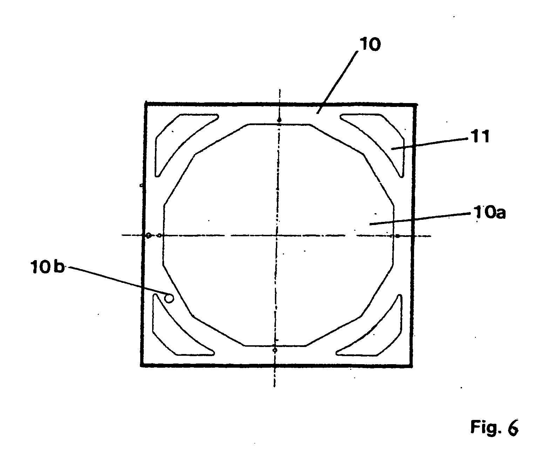

Figur 6 zeigt eine Ansaugplatte 10 inklusive vier Ansaugöffnungen 11 und einer Aussparung 10a für einen zwölfeckigen Reflektor. Außerdem ist eine Öffnung 10b mit Gewindedurchzug vorhanden, durch welche ein Drucktaster auf einen Strahlungsfilter aufsetzen kann.

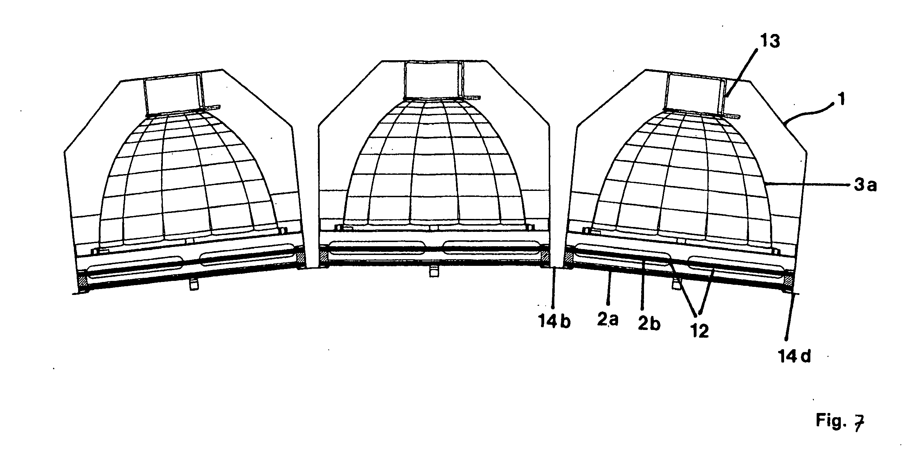

Figur 7 zeigt drei Bräunungsmodule mit einem Gehäuse gemäß Figur 1 im Schnitt quer zum Dachfirst, wobei die Anordnung den Anschluss der Falze 14b, 14d von Modul zu Modul zeigt.

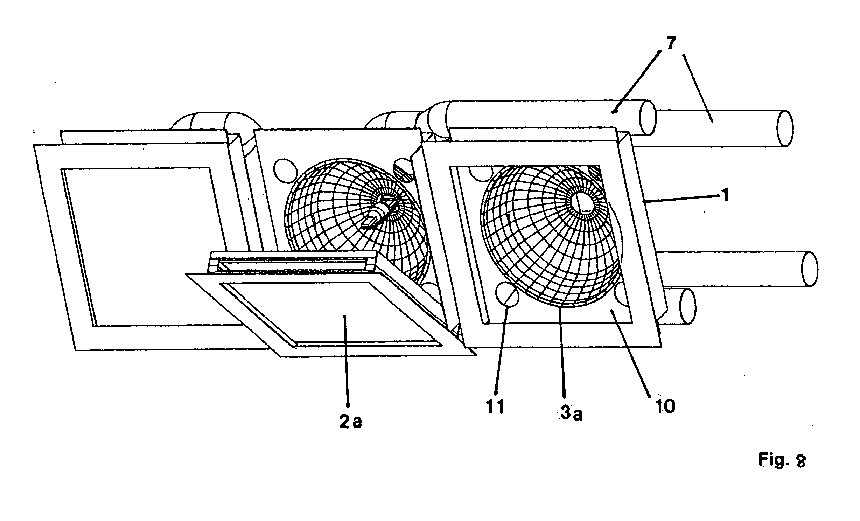

Figur 8 zeigt eine dreidimensionale Ansicht mehrerer nebeneinander montierter Bräunungsmodule. Ganz links ist ein Bräunungsmodul mit geschlossenem Strahlungsfilter gezeigt. In der Mitte ist ein Bräunungsmodul mit aufgeklapptem Strahlungsfilter 2a inklusive Bräunungsstrahler abgebildet, wobei die Ansaugplatte 10 mit kreisrunden Ansaugöffnungen 11 sowie der Reflektor 3a erkennbar ist. Ganz rechts ist ein Bräunungsmodul ohne Strahlungsfilter und ohne Bräunungsstrahler gezeigt. Alle drei Module sind mit Luftabsaugschläuchen 7 versehen.

## Patentansprüche

1. Bräunungsmodul mit einem Gehäuse, einem im Gehäuse (1) angeordneten, dreidimensionalen Reflektor (3a, 3b) sowie mindestens einem scheibenförmigen Strahlungsfilter (2a, 2b), wobei der mindestens eine Strahlungsfilter (2a, 2b) die Strahlungsaustrittsfläche des Reflektors (3a, 3b) überdeckt und an einer ersten Seite des Gehäuses (1) angeordnet ist, wobei im Reflektor (3a, 3b) mindestens eine Öffnung zum Einbau und elektrischen Anschluss eines Bräunungsstrahlers vorgesehen ist, und wobei der Reflektor im Bereich der Strahlungsaustrittsfläche seinen maximalen Querschnitt aufweist, **dadurch gekennzeichnet, dass** das Gehäuse (1) an einer dem Strahlungsfilter (2a, 2b) entgegengesetzten zweiten Seite in Form eines Walmdaches oder eines Krüppelwalmdaches (1b) ausgestaltet ist, wobei der Dachfirst (1a) abgeflacht ist und vom Strahlungsfilter (2a, 2b) wegzeigt.

2. Bräunungsmodul nach Anspruch 1, **dadurch gekennzeichnet, dass** der Strahlungsfilter (2a, 2b) parallel zur Strahlungsaustrittsfläche des Reflektors (3a, 3b) ausgerichtet ist.

3. Bräunungsmodul nach einem der Ansprüche 1 bis 2, **dadurch gekennzeichnet, dass** der abgeflachte Dachfirst (1a) durch einen planen Gehäusewandungsteil gebildet ist.

4. Bräunungsmodul nach Anspruch 3, **dadurch gekennzeichnet, dass** der plane Gehäusewandungsteil parallel zum Strahlungsfilter (2a, 2b) ausgerichtet ist.

5. Bräunungsmodul nach einem der Ansprüche 1 bis 2, **dadurch gekennzeichnet, dass** der abgeflachte Dachfirst (1a) durch einen gewölbten Gehäusewandungsteil gebildet ist.

6. Bräunungsmodul nach Anspruch 5, **dadurch gekennzeichnet, dass** der gewölbte Gehäusewandungsteil im Hinblick auf den Strahlungsfilter (2a, 2b) konkav oder konvex gewölbt ausgestaltet ist.

7. Bräunungsmodul nach einem der Ansprüche 1 bis 6, **dadurch gekennzeichnet, dass** an das Walmdach oder das Krüppelwalmdach (1b) in Richtung des Strahlungsfilters (2a, 2b) ein rechteckiger Gehäusewandungsbereich (1d) anschließt.

8. Bräunungsmodul nach einem der Ansprüche 1 bis 7, **dadurch gekennzeichnet, dass** der Reflektor (3a, 3b) becher- oder wannenförmig ausgebildet ist.

9. Bräunungsmodul nach Anspruch 8, **dadurch gekennzeichnet, dass** der Becher- oder Wannenboden des Reflektors (3a, 3b) gewölbt ist.

10. Bräunungsmodul nach Anspruch 8, **dadurch gekennzeichnet, dass** der Becher- oder Wannenboden des Reflektors (3a, 3b) planparallel zum mindestens einen Strahlungsfilter (2a, 2b) ausgeführt ist.

11. Bräunungsmodul nach einem der Ansprüche 1 bis 10, **dadurch gekennzeichnet, dass** ein Umfang des Reflektors (3a, 3b) parallel zur Strahlungsaustrittsfläche einen Kreis, eine Ellipse, ein Rechteck oder ein Vieleck beschreibt.

12. Bräunungsmodul nach Anspruch 11, **dadurch gekennzeichnet, dass** der Reflektor (3a, 3b) aus Facetten (4) gebildet ist und der Umfang des Reflektors (3a, 3b) parallel zur Strahlungsaustrittsfläche ein Vieleck mit zwölf Ecken beschreibt.

13. Bräunungsmodul nach Anspruch 12, **dadurch gekennzeichnet, dass** der Reflektor (3a, 3b) eine Höhe von 90mm bis 95mm, insbesondere von 93,6mm, und das Zwölfeck in der Ebene der Strahlungsaustrittsfläche einen maximalen Durchmesser im Bereich von 210mm bis 230mm, insbesondere von 210mm, aufweist.

14. Bräunungsmodul nach Anspruch 12, **dadurch gekennzeichnet, dass** der Reflektor (3a, 3b) eine Höhe im Bereich von 110mm bis 125mm, insbesondere von 118,7mm, und das Zwölfeck in der Ebene der Strahlungsaustrittsfläche einen maximalen Durchmesser im Bereich von 170mm bis 200mm, insbesondere von 184mm, aufweist.

15. Bräunungsmodul nach Anspruch 12, **dadurch gekennzeichnet, dass** der Reflektor (3a, 3b) eine Höhe im Bereich von 75mm bis 90mm, insbesondere von 83,3mm, und das Zwölfeck in der Ebene der Strahlungsaustrittsfläche einen maximalen Durchmesser im Bereich von 205mm bis 235mm, insbesondere von 220mm, aufweist.

16. Bräunungsmodul nach einem der Ansprüche 1 bis 15, **dadurch gekennzeichnet, dass** das Gehäuse (1) im Bereich der Dachschrägen mindestens eine Luftabsaugöffnung (5) aufweist.

17. Bräunungsmodul nach Anspruch 16, **dadurch gekennzeichnet, dass** an der mindestens einen Luftabsaugöffnung (5) ein Flansch (6) angebracht ist.

18. Bräunungsmodul nach Anspruch 17, **dadurch gekennzeichnet, dass** an dem Flansch (6) ein Luftabsaugschlauch (7) angeordnet ist.

19. Bräunungsmodul nach einem der Ansprüche 16 bis 18, **dadurch gekennzeichnet, dass** eine Reduzierscheibe zur Verkleinerung der Luftabsaugöffnung (5) vorhanden ist.

20. Bräunungsmodul nach einem der Ansprüche 1 bis 19, **dadurch gekennzeichnet, dass** außen am Gehäuse (1) mindestens eine Halterung (8) für elektrische Anschlüsse oder Komponenten (9) angeordnet ist.

21. Bräunungsmodul nach einem der Ansprüche 1 bis 20, **dadurch gekennzeichnet, dass** eine Ansaugplatte (10) zwischen Gehäuse (1) und Reflektor (3a, 3b) angeordnet ist, wobei die Strahlungsäustrittsfläche des Reflektors (3a, 3b) zur Ebene der Ansaugplatte (10) nach oben oder unten verschoben ist, wobei mindestens eine Ansaugöffnung zwischen Ansaugplatte (10) und Reflektor (3a, 3b) ausgebildet ist und die Ansaugplatte (10) eine Aussparung (10a) für den Reflektor (3a, 3b) aufweist, die in der senkrechten Projektion auf den mindestens einen Strahlungsfilter (2a, 2b) die Größe der Strahlungsaustrittsfläche des Reflektors (3a, 3b) aufweist.

22. Bräunungsmodul nach einem der Ansprüche 1 bis 20, **dadurch gekennzeichnet, dass** eine Ansaugplatte (10) das Gehäuse (1) und den Reflektor (3a, 3b) allseitig im Bereich der Strahlungsaustrittsfläche des Reflektors (3a, 3b) verbindet, wobei die Ansaugplatte (10) mindestens eine Ansaugöffnung (11) aufweist und zudem eine Aussparung (10a) für den Reflektor aufweist, die in der senkrechten Projektion auf den mindestens einen Strahlungsfilter (2a, 2b) die Größe der Strahlungsaustrittsfläche des Reflektors (3a, 3b) aufweist.

23. Bräunungsmodul nach Anspruch 22, **dadurch gekennzeichnet, dass** die Ansaugplatte (10) einen rechteckigen Umfang aufweist, dass der Umfang des Reflektors (3a, 3b) parallel zur Strahlungsaustrittsfläche einen Kreis, eine Ellipse oder ein Vieleck beschreibt und dass die mindestens eine Ansaugöffnung (11) im Bereich einer Ecke der Ansaugplatte (10) angeordnet ist.

24. Bräunungsmodul nach Anspruch 23, **dadurch gekennzeichnet, dass** vier Ansaugöffnungen (11) in der Ansaugplatte (10) ausgebildet sind und dass je eine Ansaugöffnung (11) in je einer Ecke der Ansaugplatte (10) angeordnet ist.

25. Bräunungsmodul nach einem der Ansprüche 23 bis 24, **dadurch gekennzeichnet, dass** die mindestens eine Ansaugöffnung (11) längs der Seiten der Ansaugplatte (10) vergrößert ist.

26. Bräunungsmodul nach Anspruch 25, **dadurch gekennzeichnet, dass** die Ansaugöffnung (11) trapezförmig ist, wobei die lange Seite des Trapezes zum Reflektor (3a, 3b) zeigt.

27. Bräunungsmodul nach Anspruch 26, **dadurch gekennzeichnet, dass** die lange Seite des Trapezes sowie deren gegenüberliegende Seite gekrümmt sind.

28. Bräunungsmodul nach einem der Ansprüche 21 bis 27, **dadurch gekennzeichnet, dass** der Reflektor (3a, 3b) nur über die Ansaugplatte (10) am Gehäuse (1) befestigt ist.

29. Bräunungsmodul nach einem der Ansprüche 1 bis 28, **dadurch gekennzeichnet, dass** der mindestens eine Strahlungsfilter (2a, 2b) über einen Schwenkmechanismus vom Gehäuse (1) lösbar ist.

30. Bräunungsmodul nach einem der Ansprüche 1 bis 29, **dadurch gekennzeichnet, dass** der mindestens eine Strahlungsfilter (2a, 2b) rechteckig ausgebildet ist.

31. Bräunungsmodul nach Anspruch 30, **dadurch gekennzeichnet, dass** der mindestens eine Strahlungsfilter (2a, 2b) eine Länge und eine Breite im Bereich von 215mm bis 240mm aufweist.

32. Bräunungsmodul nach Anspruch 31, **dadurch gekennzeichnet, dass** der mindestens eine Strahlungsfilter (2a, 2b) eine Länge von 230mm und eine Breite von 225mm aufweist.

33. Bräunungsmodul nach einem der Ansprüche 1 bis 32, **dadurch gekennzeichnet, dass** der mindestens eine Strahlungsfilter (2a, 2b) ein Interferenzfilter ist.

34. Bräunungsmodul nach einem der Ansprüche 1 bis 33, **dadurch gekennzeichnet, dass** mindestens eine Luftansaugöffnung (12) im Bereich des Umfanges des mindestens einen Strahlungsfilters (2a, 2b) im Gehäuse (1) und/oder zwischen Gehäuse (1) und Strahlungsfilter (2a, 2b) vorhanden ist.

35. Bräunungsmodul nach einem der Ansprüche 33 bis 34, **dadurch gekennzeichnet, dass** ein erster Strahlungsfilter (2a) und planparallel dazu ein zweiter Strahlungsfilter (2b) vorhanden ist, wobei der zweite Strahlungsfilter (2b) zwischen der Strahlungsaustrittsfläche des Reflektors (3a, 3b) und dem ersten Strahlungsfilter (2a) angeordnet ist und wobei der erste Strahlungsfilter (2a) der Interferenzfilter ist.

36. Bräunungsmodul nach Anspruch 35, **dadurch gekennzeichnet, dass** der zweite Strahlungsfilter (2b) ein UV-Filter oder ein Infrarotfilter ist.

37. Bräunungsmodul nach einem der Ansprüche 1 bis 36, **dadurch gekennzeichnet, dass** zur Bruchsicherung des mindestens einen Strahlungsfilters (2a, 2b) mindestens ein Drucktaster am Gehäuse (1) angeordnet ist, der auf den mindestens einen Strahlungsfilter (2a, 2b) aufsetzt.

38. Bräunungsmodul nach Anspruch 37, **dadurch gekennzeichnet, dass** der Drucktaster senkrecht zur Strahlungsaustrittsfläche des Reflektors (3a, 3b) durch den Reflektor (3a, 3b) geführt ist.

39. Bräunungsmodul nach Anspruch 37, **dadurch gekennzeichnet, dass** der Drucktaster senkrecht zur Strahlungsaustrittsfläche des Reflektors (3a, 3b) durch die Ansaugplatte (10) geführt ist.

40. Bräunungsmodul nach einem der Ansprüche 1 bis 39, **dadurch gekennzeichnet, dass** ein Sockel (13) im Bereich der Öffnung des Reflektors (3a, 3b) zum mechanischen und elektrischen Anschluss eines Bräunungsstrahlers vorgesehen ist.

41. Bräunungsmodul nach einem der Ansprüche 21 bis 40, **dadurch gekennzeichnet, dass** zwischen dem mindestens einen Strahlungsfilter (2a, 2b) und der Ansaugplatte (10) eine Abdeckplatte angeordnet ist, die von der Ansaugplatte (10) beabstandet angeordnet ist und welche eine Aussparung aufweist, die in der senkrechten Projektion auf den mindestens einen Strahlungsfilter die Größe der Strahlungsaustrittsfläche des Reflektors (3a, 3b) aufweist.

42. Bräunungsmodul nach einem der Ansprüche 1 bis 41, **dadurch gekennzeichnet, dass** das Gehäuse (1) im Bereich des Strahlungsfilters (2a, 2b) rechteckig ist und an allen vier Seiten mit jeweils einem Falz (14a, 14b, 14c, 14d) abschließt, wobei die vier Falze (14a, 14b, 14c, 14d) parallel zum Strahlungsfilter (2a, 2b) ausgerichtet sind.

43. Bräunungsmodul nach Anspruch 42, **dadurch gekennzeichnet, dass** zwei sich gegenüberliegende Falze (14a, 14c) der vier Falze (14a, 14b, 14c, 14d) zum Strahlungsfilter (2a, 2b) zeigen.

44. Bräunungsmodul nach Anspruch 43, **dadurch gekennzeichnet, dass** die übrigen zwei sich gegenüberliegenden Falze (14b, 14d) der vier Falze (14a, 14b, 14c, 14d) vom Strahlungsfilter (2a, 2b) weg zeigen.

## Claims

1. Tanning module with a housing, a three-dimensional reflector (3a, 3b) arranged in the housing (1) and at least one disc-shaped radiation filter (2a, 2b), wherein the at least one radiation filter (2a, 2b) covers the radiation outlet face of the reflector (3a, 3b) and is arranged on a first side of the housing (1), wherein at least one opening is provided in the reflector (3a, 3b) for the insertion and electrical connection of a tanning radiator, and wherein the reflector has its maximum cross-section in the area of the radiation outlet face, **characterised in that** the housing (1) is configured on a second side opposing the radiation filter (2a, 2b) in the shape of a hip roof or a false hip roof (1b), the roof ridge (1a) being flattened and pointing away from the radiation filter (2a, 2b).

2. Tanning module according to claim 1, **characterised in that** the radiation filter (2a, 2b) is aligned parallel to the radiation outlet face of the reflector (3a, 3b).

3. Tanning module according to either of claims 1 or 2, **characterised in that** the flattened roof ridge (1 a) is formed by a planar housing wall part.

4. Tanning module according to claim 3, **characterised in that** the planar housing wall part is aligned parallel to the radiation filter (2a, 2b).

5. Tanning module according to either of claims 1 or 2, **characterised in that** the flattened roof ridge (1 a) is formed by an arched housing wall part.

6. Tanning module according to claim 5, **characterised in that** the arched housing wall part is configured concavely or convexly arched with respect to the radiation filter (2a, 2b).

7. Tanning module according to any one of claims 1 to 6, **characterised in that** a rectangular housing wall area (1d) adjoins the hip roof or false hip roof (1b) in the direction of the radiation filter (2a, 2b).

8. Tanning module according to any one of claims 1 to 7, **characterised in that** the reflector (3a, 3b) is configured as a cup or trough shape.

9. Tanning module according to claim 8, **characterised in that** the cup or trough base of the reflector (3a, 3b) is arched.

10. Tanning module according to claim 8, **characterised in that** the cup or trough base of the reflector (3a, 3b) is designed plane-parallel to the at least one radiation filter (2a, 2b).

11. Tanning module according to any one of claims 1 to 10, **characterised in that** a periphery of the reflector (3a, 3b) parallel to the radiation outlet face describes a circle, an ellipse, a rectangle or a polygon.

12. Tanning module according to claim 11, **characterised in that** the reflector (3a, 3b) is formed from facets (4) and the periphery of the reflector (3a, 3b) parallel to the radiation outlet face, describes a polygon with twelve corners.

13. Tanning module according to claim 12, **characterised in that** the reflector (3a, 3b) has a height of 90 mm to 95 mm, in particular 93.6 mm, and the dodecagon has a maximum diameter in the plane of the radiation outlet face ranging from 210 mm to 230 mm, in particular 210 mm.

14. Tanning module according to claim 12, **characterised in that** the reflector (3a, 3b) has a height ranging from 110 mm to 125 mm, in particular 118.7 mm, and the dodecagon has, in the plane of the radiation outlet face, a maximum diameter ranging from 170 mm to 200 mm, in particular 184 mm.

15. Tanning module according to claim 12, **characterised in that** the reflector (3a, 3b) has a height ranging from 75 mm to 90 mm, in particular 83.3 mm, and the dodecagon, in the plane of the radiation outlet face, has a maximum diameter ranging from 205 mm to 235 mm, in particular 220 mm.

16. Tanning module according to any one of claims 1 to 15, **characterised in that** the housing (1) has, in the area of the roof slopes, at least one air exhaust opening (5).

17. Tanning module according to claim 16, **characterised in that** a flange (6) is applied to the at least one air exhaust opening (5).

18. Tanning module according to claim 17, **characterised in that** an air exhaust tube (7) is arranged on the flange (6).

19. Tanning module according to any one of claims 16 to 18, **characterised in that** a reducing disc is present to reduce the size of the air exhaust opening (5).

20. Tanning module according to any one of claims 1 to 19, **characterised in that** at least one mount (8) for electrical connections or components (9) is arranged on the outside of the housing (1).

21. Tanning module according to any one of claims 1 to 20, **characterised in that** an intake plate (10) is arranged between the housing (1) and reflector (3a, 3b), the radiation outlet face of the reflector (3a, 3b) being displaced upward or downward with respect to the plane of the intake plate (10), at least one intake opening being formed between the intake plate (10) and the reflector (3a, 3b) and the intake plate (10) having a recess (10a) for the reflector (3a, 3b), which, in its vertical projection onto the at least one radiation filter (2a, 2b), has the size of the radiation outlet face of the reflector (3a, 3b).

22. Tanning module according to any one of claims 1 to 20, **characterised in that** an intake plate (10) connects the housing (1) and the reflector (3a, 3b) on all sides in the area of the radiation outlet face of the reflector (3a, 3b), the intake plate (10) having at least one intake opening (11) and also a recess (10a) for the reflector, which in vertical projection onto the at least one radiation filter (2a, 2b), has the size of the radiation outlet face of the reflector (3a, 3b).

23. Tanning module according to claim 22, **characterised in that** the intake plate (10) has a rectangular periphery, **in that** the periphery of the reflector (3a, 3b) parallel to the radiation outlet face describes a circle, an ellipse or a polygon, and **in that** the at least one intake opening (11) is arranged in the area of a corner of the intake plate (10).

24. Tanning module according to claim 23, **characterised in that** four intake openings (11) are formed in the intake plate (10) and **in that** an intake opening (11) is arranged in each corner of the intake plate (10).

25. Tanning module according to claim 23 or 24, **characterised in that** the at least intake opening (11) is enlarged along the sides of the intake plate (10).

26. Tanning module according to claim 25, **characterised in that** the intake opening (11) is trapezoidal, the long side of the trapezium pointing to the reflector (3a, 3b).

27. Tanning module according to claim 26, **characterised in that** the long side of the trapezium and its opposite side are curved.

28. Tanning module according to any one of claims 21 to 27, **characterised in that** the reflector (3a, 3b) is fastened to the housing (1) only by the intake plate (10).

29. Tanning module according to any one of claims 1 to 28, **characterised in that** the at least one radiation filter (2a, 2b) is releasable from the housing (1) by a swing mechanism.

30. Tanning module according to any one of claims 1 to 29, **characterised in that** the at least one radiation filter (2a, 2b) is of rectangular configuration.

31. Tanning module according to claim 30, **characterised in that** the at least one radiation filter (2a, 2b) has a length and a width ranging from 215 mm to 240 mm.

32. Tanning module according to claim 31, **characterised in** the at least one radiation filter (2a, 2b) has a length of 230 mm and a width of 225 mm.

33. Tanning module according to any one of claims 1 to 32, **characterised in that** the at least one radiation filter (2a, 2b) is an interference filter.

34. Tanning module according to any one of claims 1 to 33, **characterised in that** at least one air intake opening (12) is present in the area of the periphery of the at least one radiation filter (2a, 2b) in the housing (1) and/or between the housing (1) and the radiation filter (2a, 2b).

35. Tanning module according to any one of claims 33 to 34, **characterised in that** a first radiation filter (2a) is present, and plane-parallel thereto, a second radiation filter (2b), the second radiation filter (2b) being arranged between the radiation outlet face of the reflector (3a, 3b) and wherein the first radiation filter (2a), and the first radiation filter (2a) is the interference filter.

36. Tanning module according to claim 35, **characterised in that** the second radiation filter (2b) is a UV filter or an infrared filter.

37. Tanning module according to any one of claims 1 to 36, **characterised in that** to safeguard against breakage of the at least one radiation filter (2a, 2b), at least one pressure switch is arranged on the housing (1) and rests on the at least one radiation filter (2a, 2b).

38. Tanning module according to claim 37, **characterised in that** the pressure switch is guided through the reflector (3a, 3b) perpendicular to the radiation outlet face of the reflector (3a, 3b).

39. Tanning module according to claim 37, **characterised in that** the pressure switch is guided perpendicular to the radiation outlet face of the reflector (3a, 3b) through the intake plate (10).

40. Tanning module according to any one of claims 1 to 39, **characterised in that** a socket (13) is provided in the area of the opening of the reflector (3a, 3b) for the mechanical and electrical connection of a tanning radiator.

41. Tanning module according to any one of claims 21 to 40, **characterised in that** between the at least one radiation filter (2a, 2b) and the intake plate (10), a cover plate is arranged, which is spaced apart from the intake plate (10) and which has a recess which, in its vertical projection onto the at least one radiation filter, has the size of the radiation outlet face of the reflector (3a, 3b).

42. Tanning module according to any one of claims 1 to 41, **characterised in that** the housing (1) is rectangular in the area of the radiation filter (2a, 2b) and on all four sides terminates in a lip (14a, 14b, 14c, 14d), the four lips (14a, 14b, 14c, 14d) being aligned parallel to the radiation filter (2a, 2b).

43. Tanning module according to claim 42, **characterised in that** two opposing lips (14a, 14c) of the four lips (14a, 14b, 14c, 14d) point toward the radiation filter (2a, 2b).

44. Tanning module according to claim 43, **characterised in that** the other two opposing lips (14b, 14d) of the four lips (14a, 14b, 14c, 14d) point away from the radiation filter (2a, 2b).

## Revendications

1. Module de bronzage comprenant un boîtier, un réflecteur tridimensionnel (3a, 3b) agencé dans le boîtier (1), et au moins un filtre de rayonnement (2a, 2b) en forme de plaque, ledit au moins un filtre de rayonnement (2a, 2b) recouvrant la surface de sortie de rayonnement du réflecteur (3a, 3b) et étant agencé sur un côté du boîtier (1), dans lequel au moins une ouverture pour le montage et pour le raccordement électrique d'un élément rayonneur de bronzage est ménagée dans le réflecteur (3a, 3b), et le réflecteur présente sa section maximale dans la zone de la surface de sortie de rayonnement, **caractérisé en ce que** le boîtier (1) est réalisé, sur son second côté opposé au filtre de rayonnement (2a, 2b), sous la forme d'une toiture en croupe ou d'une toiture en demi-croupe (1b), dans laquelle le faîte de toiture (1a) est aplati et dirigé à l'opposé du filtre de rayonnement (2a, 2b).

2. Module de bronzage selon la revendication 1, **caractérisé en ce que** le filtre de rayonnement (2a, 2b) est orienté parallèlement à la surface de sortie de rayonnement du réflecteur (3a, 3b).

3. Module de bronzage selon l'une des revendications 1 et 2, **caractérisé en ce que** le faîte de toiture aplati (1a) est formé par une partie plane de la paroi du boîtier.

4. Module de bronzage selon la revendication 3, **caractérisé en ce que** la partie plane de la paroi de boîtier est orientée parallèlement au filtre de rayonnement (2a, 2b).

5. Module de bronzage selon l'une des revendications 1 et 2, **caractérisé en ce que** le faîte de toiture aplati (1a) est formé par une partie bombée de la paroi de boîtier.

6. Module de bronzage selon la revendication 5, **caractérisé en ce que** la partie bombée de la paroi de boîtier est réalisée avec un bombement concave ou convexe par rapport au filtre de rayonnement (2a, 2b).

7. Module de bronzage selon l'une des revendications 1 à 6, **caractérisé en ce qu'**une zone rectangulaire de la paroi du boîtier (1d) se raccorde à la toiture en croupe ou à la toiture en demi-croupe (1b) en direction du filtre de rayonnement (2a, 2b).

8. Module de bronzage selon l'une des revendications 1 à 7, **caractérisé en ce que** le réflecteur (3a, 3b) est réalisé en forme de godet ou en forme de cuve.

9. Module de bronzage selon la revendication 8, **caractérisé en ce que** le fond en godet ou en cuve du réflecteur (3a, 3b) est bombé.

10. Module de bronzage selon la revendication 8, **caractérisé en ce que** le fond en godet ou en cuve du réflecteur (3a, 3b) est réalisé parallèle au plan dudit au moins un filtre de rayonnement (2a, 2b).

11. Module de bronzage selon l'une des revendications 1 à 10, **caractérisé en ce qu'**une circonférence du réflecteur (3a, 3b) parallèle à la surface de sortie de rayonnement décrit un cercle, une ellipse, un rectangle ou un polygone.

12. Module de bronzage selon la revendication 11, **caractérisé en ce que** le réflecteur (3a, 3b) est formé de facettes (4), et la circonférence du réflecteur (3a, 3b) parallèle à la surface de sortie de rayonnement décrit un polygone à 12 côtés.

13. Module de bronzage selon la revendication 12, **caractérisé en ce que** le réflecteur (3a, 3b) présente une hauteur de 90 mm à 95 mm, en particulier de 93,6 mm, et le dodécagone présente, dans le plan de la surface de sortie de rayonnement, un diamètre maximum dans la plage de 210 mm à 230 mm, en particulier de 210 mm.

14. Module de bronzage selon la revendication 12, **caractérisé en ce que** le réflecteur (3a, 3b) présente une hauteur de 110 mm à 125 mm, en particulier de 118,7 mm, et le dodécagone présente, dans le plan de la surface de sortie de rayonnement, un diamètre maximum dans la plage de 170 mm à 200 mm, en particulier de 184 mm.

15. Module de bronzage selon la revendication 12, **caractérisé en ce que** le réflecteur (3a, 3b) présente une hauteur dans la plage de 75 à 90 mm, en particulier de 83,3 mm, et le dodécagone présente, dans le plan de la surface de sortie de rayonnement, un diamètre maximum dans la plage de 205 mm à 235 mm, en particulier de 220 mm.

16. Module de bronzage selon l'une des revendications 1 à 15, **caractérisé en ce que** le boîtier (1) comprend au moins une ouverture d'aspiration d'air (5) dans la zone des pentes de toiture.

17. Module de bronzage selon la revendication 16, **caractérisé en ce qu'**une bride (6) est montée sur ladite au moins une ouverture d'aspiration d'air (5).

18. Module de bronzage selon la revendication 17, **caractérisé en ce qu'**un tuyau d'aspiration d'air (7) et agencé sur la bride (6).

19. Module de bronzage selon l'une des revendications 16 à 18, **caractérisé en ce qu'**une plaque de réduction est prévue pour réduire l'ouverture d'aspiration d'air (5).

20. Module de bronzage selon l'une des revendications 1 à 19, **caractérisé en ce qu'**au moins une monture (8) pour des bornes électriques ou pour des composants électriques (9) est agencée à l'extérieur sur le boîtier (1).

21. Module de bronzage selon l'une des revendications 1 à 20, **caractérisé en ce qu'**une plaque d'aspiration (10) est agencée entre le boîtier (1) et le réflecteur (3a, 3b), la surface de sortie de rayonnement du réflecteur (3a, 3b) est déplacée vers le haut ou vers le bas vers le plan de la plaque d'aspiration (10), au moins une ouverture d'aspiration (11) est réalisée entre la plaque d'aspiration (10) et le réflecteur (3a, 3b) et la plaque d'aspiration (10) présente une échancrure (10a) pour le réflecteur (3a, 3b) qui, en projection perpendiculaire sur ledit au moins un filtre de rayonnement (2a, 2b), a la taille de la surface de sortie de rayonnement du réflecteur (3a, 3b).

22. Module de bronzage selon l'une des revendications 1 à 20, **caractérisé en ce qu'**une plaque d'aspiration (10) relie le boîtier (1) et le réflecteur (3a, 3b) sur tous les côtés dans la zone de la surface de sortie de rayonnement du réflecteur (3a, 3b), ladite plaque d'aspiration (10) présentant au moins une ouverture d'aspiration (11), et présentant en outre une échancrure (10a) pour le réflecteur qui, en projection perpendiculaire sur ledit au moins un filtre de rayonnement (2a, 2b), a la taille de la surface de sortie de rayonnement du réflecteur (3a, 3b).

23. Module de bronzage selon la revendication 22, **caractérisé en ce que** la plaque d'aspiration (10) présente un périmètre rectangulaire, **en ce que** le périmètre du réflecteur (3a, 3b) parallèle à la surface de sortie de rayonnement décrit un cercle, une ellipse ou un polygone, et **en ce que** ladite au moins une ouverture d'aspiration (11) est agencée dans la zone d'un coin de la plaque d'aspiration (10).

24. Module de bronzage selon la revendication 23, **caractérisé en ce que** quatre ouvertures d'aspiration (11) sont ménagées dans la plaque d'aspiration (10), et **en ce que** chaque ouverture d'aspiration (11) est agencée dans un coin respectif de la plaque d'aspiration (10).

25. Module de bronzage selon l'une des revendications 23 et 24, **caractérisé en ce que** ladite au moins une ouverture d'aspiration (11) est agrandie le long des côtés de la plaque d'aspiration (10).

26. Module de bronzage selon la revendication 25, **caractérisé en ce que** l'ouverture d'aspiration (11) est en forme de trapèze, le grand côté du trapèze étant dirigé vers le réflecteur (3a, 3b).

27. Module de bronzage selon la revendication 26, **caractérisé en ce que** le grand côté du trapèze ainsi que son côté opposé sont incurvés.

28. Module de bronzage selon l'une des revendications 21 à 27, **caractérisé en ce que** le réflecteur (3a, 3b) est fixé sur le boîtier (1) uniquement via la plaque d'aspiration (10).

29. Module de bronzage selon l'une des revendications 1 à 28, **caractérisé en ce que** ledit au moins un filtre de rayonnement (2a, 2b) est détachable du boîtier (1) via un mécanisme de pivotement.

30. Module de bronzage selon l'une des revendications 1 à 29, **caractérisé en ce que** ledit au moins un filtre de rayonnement (2a, 2b) est réalisé rectangulaire.

31. Module de bronzage selon la revendication 30, **caractérisé en ce que** ledit au moins un filtre de rayonnement (2a, 2b) présente une longueur et une largeur dans la plage de 215 mm à 240 mm.

32. Module de bronzage selon la revendication 31, **caractérisé en ce que** ledit au moins un filtre de rayonnement (2a, 2b) présente une longueur de 230 mm et une largeur de 225 mm.

33. Module de bronzage selon l'une des revendications 1 à 32, **caractérisé en ce que** ledit au moins un filtre de rayonnement (2a, 2b) est un filtre à interférence.

34. Module de bronzage selon l'une des revendications 1 à 33, **caractérisé en ce qu'**il est prévu au moins une ouverture d'aspiration d'air (12) dans la zone du périmètre dudit au moins un filtre de rayonnement (2a, 2b) dans le boîtier (1) et/ou entre le boîtier (1) et le filtre de rayonnement (2a, 2b).

35. Module de bronzage selon l'une des revendications 33 et 34, **caractérisé en ce qu'**il est prévu un premier filtre de rayonnement (2a) et, parallèlement au plan de celui-ci, un second filtre de rayonnement (2b), ledit second filtre de rayonnement (2b) étant agencé entre la surface de sortie de rayonnement du réflecteur (3a, 3b) et le premier filtre de rayonnement (2a), ledit premier filtre de rayonnement (2a) étant le filtre à interférence.

36. Module de bronzage selon la revendication 35, **caractérisé en ce que** le second filtre de rayonnement (2b) est un filtre à ultraviolets ou un filtre à infrarouges.

37. Module de bronzage selon l'une des revendications 1 à 36, **caractérisé en ce que** pour assurer une sécurité vis-à-vis du bris dudit au moins un filtre de rayonnement (2a, 2b), il est prévu au moins un bouton-poussoir sur le boîtier (1) qui s'applique sur ledit au moins un filtre de rayonnement (2a, 2b).

38. Module de bronzage selon la revendication 37, **caractérisé en ce que** le bouton-poussoir est guidé à travers le réflecteur (3a, 3b) perpendiculairement à la surface de sortie de rayonnement du réflecteur (3a, 3b).

39. Module de bronzage selon la revendication 37, **caractérisé en ce que** le bouton-poussoir est guidé à travers la plaque d'aspiration (10) perpendiculairement à la surface de sortie de rayonnement du réflecteur (3a, 3b).

40. Module de bronzage selon l'une des revendications 1 à 39, **caractérisé en ce qu'**il est prévu un socle (13) dans la zone de l'ouverture du réflecteur (3a, 3b) pour le raccordement mécanique et électrique d'un élément rayonneur de bronzage.

41. Module de bronzage selon l'une des revendications 21 à 40, **caractérisé en ce que**, entre le ledit au moins un filtre de rayonnement (2a, 2b) et la plaque d'aspiration (10), est agencée une plaque de recouvrement qui est agencée à distance de la plaque de respiration (10) et qui présente une échancrure qui, en projection perpendiculaire sur ledit au moins un filtre de rayonnement, présente la taille de la surface de sortie de rayonnement du réflecteur (3a, 3b).

42. Module de bronzage selon l'une des revendications 1 à 41, **caractérisé en ce que** le boîtier (1) est rectangulaire dans la zone du filtre de rayonnement (2a, 2b) et se termine sur tous les quatre côtés par une feuillure respective (14a, 14b, 14c, 14d), les quatre feuillures (14a, 14b, 14c, 14d) étant orientées parallèlement au filtre de rayonnement (2a, 2b).

43. Module de bronzage selon la revendication 42, **caractérisé en ce que** deux feuillures opposées (14a, 14c) des quatre feuillures (14a, 14b, 14c, 14d) sont dirigées vers le filtre de rayonnement (2a, 2b).

44. Module de bronzage selon la revendication 43, **caractérisé en ce que** les deux autres feuillures opposées (14b, 14d) des quatre feuillures (14a, 14b, 14c, 14d) sont dirigées à l'opposé du filtre de rayonnement (2a, 2b).
